# FASCICULE DE BREVET EUROPEEN

(11) **EP 3 151 762 B1**
(45) Date de publication et mention de la délivrance du brevet: **14.02.2018**
(21) Numéro de dépôt: 15733804.7
(22) Date de dépôt: 04.06.2015
(51) Int. Cl.: A61B 17/22

(54) **DISPOSITIF DE DECOUPE ET D'EVACUATION DE TISSUS CALCIFIES D'UNE VALVE CARDIAQUE**
VORRICHTUNG ZUM ABTRENNEN UND ENTFERNEN VON VERKALKTEM GEWEBE AUS EINER HERZKLAPPE
DEVICE FOR CUTTING OFF AND REMOVING CALCIFIED TISSUE FROM A HEART VALVE

(30) Priorité: 05.06.2014 FR 1455145
(43) Date de publication de la demande: 12.04.2017
(73) Titulaire: Pain, Bernard, 43120 Monistrol-sur-Loire (FR); Vola, Marco, 42270 Saint-Priest-en Jarez (FR)
(72) Inventeur: PAIN, Bernard, 43120 Monistrol-sur-Loire (FR); VOLA, Marco, 42270 Saint-Priest-en Jarez (FR); PASQUINO, Enrico, 10020 Marentino (IT)
(74) Mandataire: Cabinet Laurent & Charras
(86) Numéro de dépôt international: PCT/FR2015/051490
(87) Numéro de publication internationale: WO 2015/185871

(56) Documents cités:
- WO-A1-2013/080729
- WO-A2-03/088809
- US-A1- 2004 049 215

## Description

L'invention concerne le domaine technique de la chirurgie endoscopique, et concerne plus particulièrement un dispositif de découpe et d'évacuation de tissus calcifiés des valves cardiaques.

Dans le domaine de la chirurgie cardiaque, il est connu, lors d'une intervention de remplacement d'une valve aortique, à coeur ouvert, c'est-à-dire en circulation extra-corporelle, que la première étape consiste à décalcifier et à enlever les parties de la valve malade susceptibles de générer des fuites para-valvulaires au niveau de la prothèse qui doit être implantée. Il est donc nécessaire de découper ces tissus malades et calcifiés. Généralement, cette opération s'effectue au moyen d'un ciseau, avec obligation de maintenir les morceaux découpés, afin d'éviter qu'ils se retrouvent perdus dans le système sanguin. Un tel geste opératoire est relativement simple en chirurgie ouverte étant donné qu'il y a un accès large au niveau de la cage thoracique qui est découpée.

Toutefois, ce type d'opération s'effectue de plus en plus par une chirurgie mini-invasive, avec des incisions de la paroi thoracique (mini-sternotomies ou mini-thoracotomies), ou éventuellement exclusivement thoracoscopique Un tel mode opératoire nécessite de prendre plus de précautions compte tenu du risque que des morceaux de tissus découpés se perdent dans le système sanguin du patient.

Le document US 2004/0049215 A1 (le préambule de la revendication 1 est basé sur ce document) décrit un dispositif de découpe et d'évacuation exemplaire. A partir de cet état de la technique, un des problèmes que se propose de résoudre l'invention, dans le cas d'une chirurgie mini-invasive, est de pouvoir découper, en un seul geste, sans extraction et réintroduction itérative dans le thorax du patient d'instruments, et d'une seule main, des tissus calcifiés de la valve native en évitant tous risques que ces tissus découpés repartent dans le système sanguin.

Pour résoudre ce problème, il a été conçu et mis au point un dispositif de découpe et d'évacuation de tissus calcifiés d'une valve cardiaque, qui est remarquable en ce qu'il comprend un corps tubulaire adapté pour être introduit par voie mini invasive ou endoscopique en relation avec un guide-fil préalablement introduit, et apte à traverser les volets de la valve au-dessus de la partie où doivent être enlevés les tissus calcifiés, l'une des extrémités dudit corps considérée au niveau desdits volets, présentant un embout profilé en matériau souple et des agencements pour un débordement latéral d'un organe de coupe desdits tissus, actionnable d'une seule main à partir d'une poignée située à l'extérieur, lesdits agencements étant en communication avec des moyens d'aspiration et d'évacuation des tissus coupés.

Il résulte de ces caractéristiques que le dispositif n'est pas obstructif au niveau de l'aorte ascendante de telle sorte que cette opération de décalcification peut être réalisée soit en chirurgie ouverte, soit en chirurgie mini-invasive et avec positionnement, parallèlement au dispositif d'optiques pour une utilisation avec vidéo- assistance ou thoracoscopie.

Pour résoudre le problème posé de pouvoir découper en un seul geste et d'une seule main les tissus calcifiés de la valve malade, l'extrémité du corps présente, en amont des agencements pour le débordement latéral de l'organe de coupe, une zone arrondie déportée latéralement pour délimiter, du côté des agencements, une partie en creux et, à l'opposé, une partie débordante apte à prendre appui avec les volets lors de l'opération de coupe sous un effet d'entraînement en rotation du corps pour réaliser la décalcification de toute la valve. Le corps est monté libre en rotation par rapport à la poignée en étant apte à être entraîné manuellement au moyen d'une molette.

Pour résoudre le problème posé de permettre au chirurgien de visualiser la zone à traiter et de vérifier, au fur et à mesure de l'intervention, les résultats obtenus, le dispositif présente un système de vision débouchant dans la partie en creux de la zone arrondie, au niveau de l'organe de coupe. Avantageusement, le corps présente un moyen apte à maintenir le système de vision en position stable pendant l'opération de coupe.

Pour résoudre le problème posé de réaliser l'ablation des tissus défectueux, les agencements pour le débordement latéral de l'organe de coupe sont constitués par une ouverture formée à l'extrémité du corps, au niveau de l'embout.

L'organe de coupe est conformé pour découper les tissus par translation et/ou rotation contre le bord de l'ouverture, depuis le centre jusqu'à la périphérie de la valve, sous un effet de pression latérale.

Dans une forme de réalisation, l'organe de coupe présente successivement des zones actives de différentes caractéristiques, pour réaliser, d'abord une coupe grossière, puis une coupe fine.

Selon une autre caractéristique, le dispositif est combiné à un système de lavage/aspiration apte à enlever les débris des tissus et de calcification dès leur ablation, ledit système étant mis en fonctionnement lorsque l'organe de coupe est actionné et obstrue l'ouverture afin de créer une étanchéité vis-à-vis de l'extérieur.

Avantageusement, l'embout souple est réalisé dans une couleur différente de la zone à décalcifier en présentant une graduation pour un positionnement précis de la zone de travail présentant l'organe de coupe.

L'invention est exposée ci-après plus en détail à l'aide des figures des dessins annexés dans lesquels :
- la figure 1 est une vue en perspective avant assemblage des principaux éléments du dispositif selon l'invention,
- la figure 2 est une vue en perspective du dispositif après montage de ses éléments constitutifs, le dispositif étant représenté en deux parties séparées avant assemblage,
- la figure 3 est une vue en perspective montrant le dispositif, le système de vision et le guide-fil,

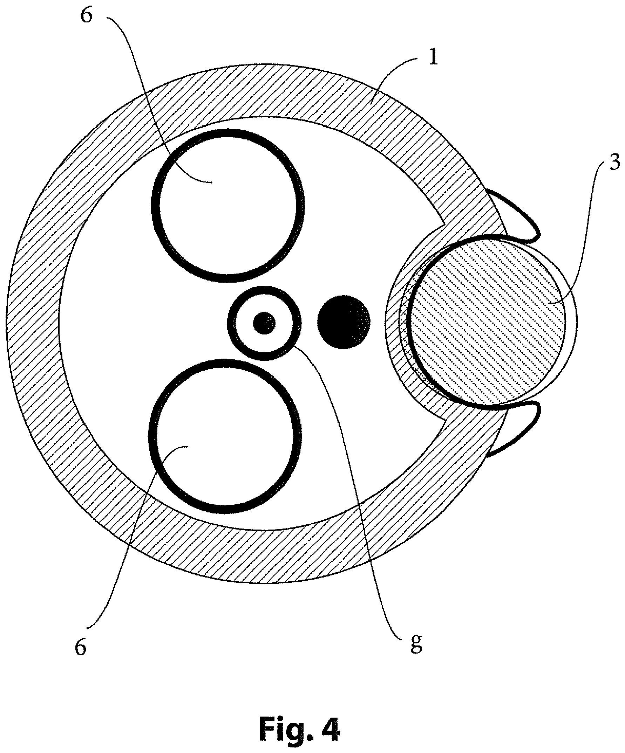

- la figure 4 est une vue en coupe transversale (A-A de la Fig. 2) à caractère schématique et à très grande échelle du dispositif,
- les figures 5 à 11 montrent les principales étapes pour l'ablation des tissus calcifiés au niveau des volets d'une valve aortique native, au moyen du dispositif selon l'invention,

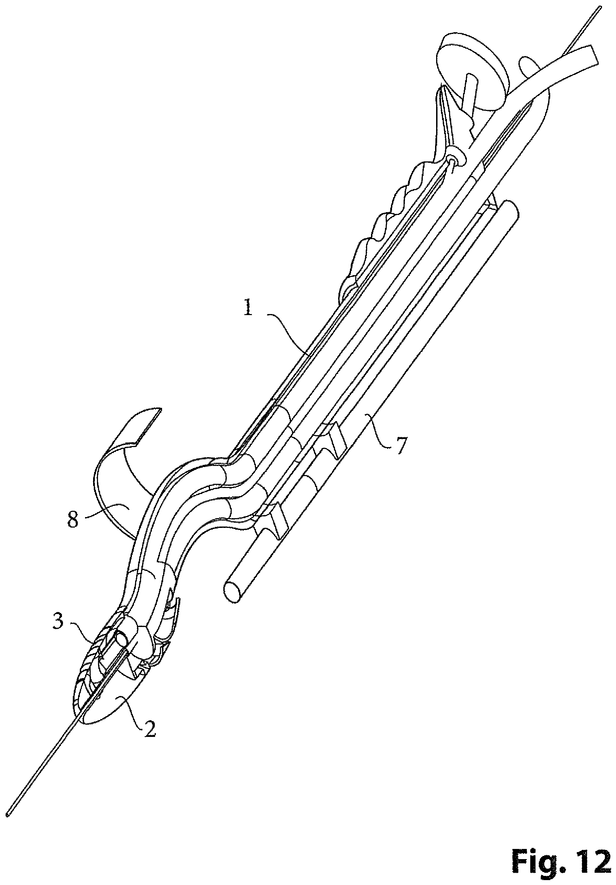

- la figure 12 est une vue en perspective avec coupe partielle du dispositif selon une forme de réalisation en variante,

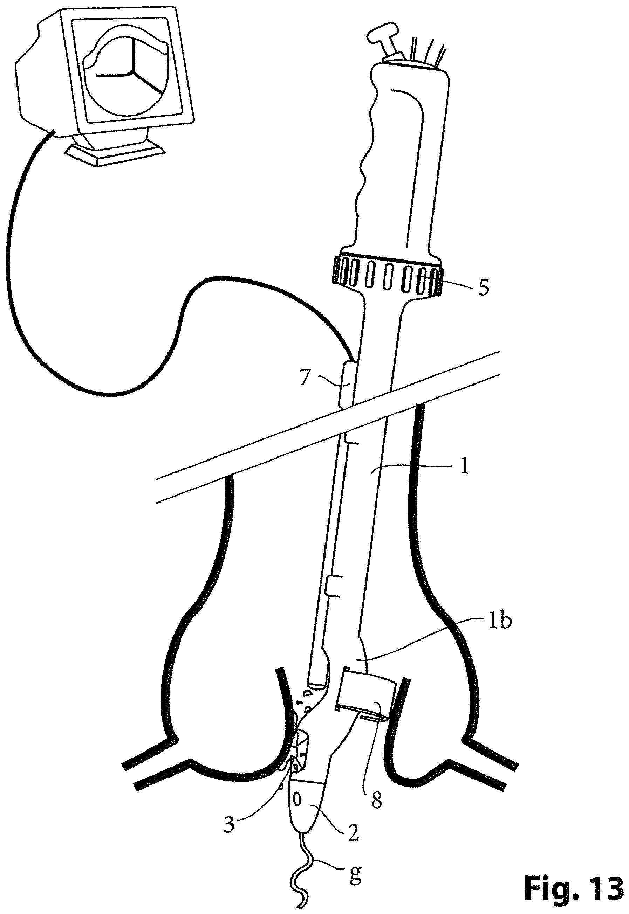

- la figure 13 montre l'utilisation du dispositif selon la forme de réalisation illustrée figure 12.

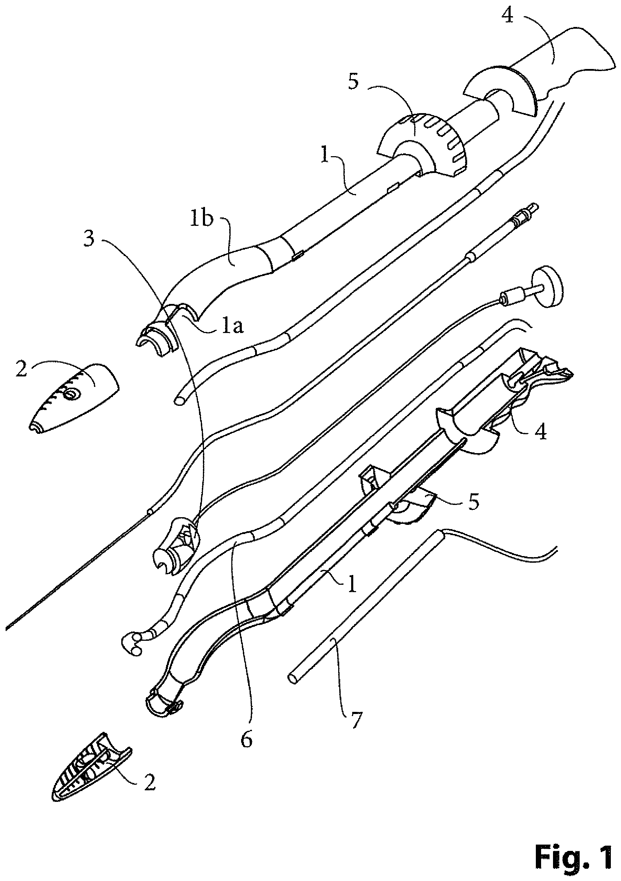

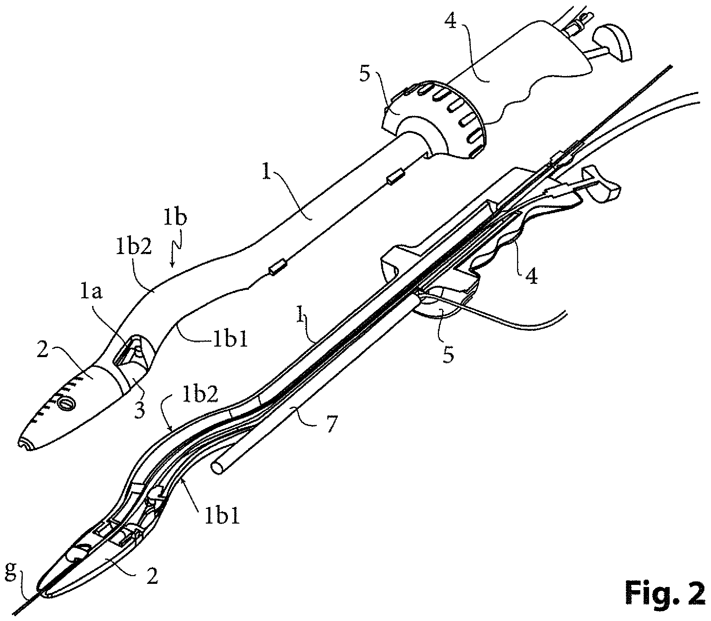

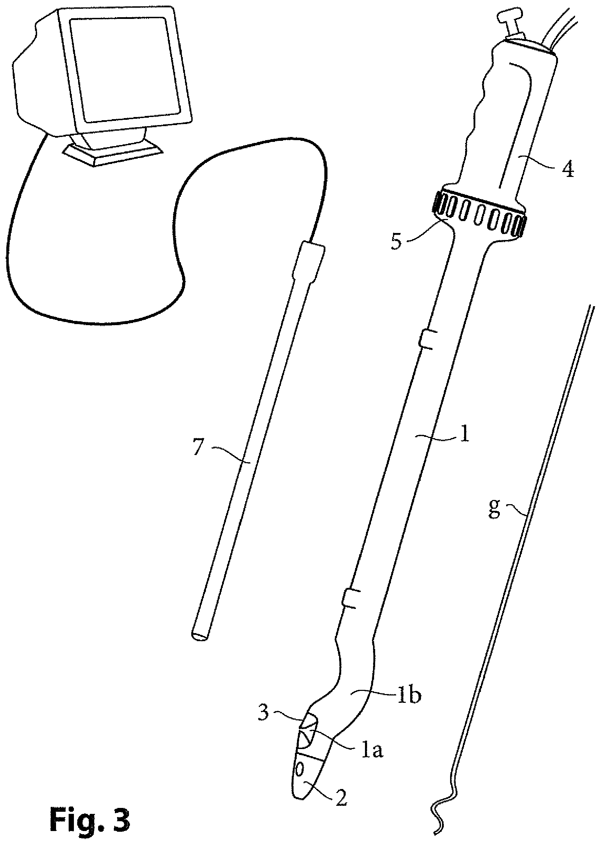

Comme le montrent notamment les figures 1, 2 et 3, le dispositif comprend un corps tubulaire (1) adapté pour être introduit par voie mini-invasive ou endoscopique, en relation avec un guide-fil (g), afin de traverser les volets de la valve native calcifiée, au-dessus de la partie où doit être enlevée, la calcification. A noter que le corps (1) peut être réalisé dans un matériau malléable afin de permettre à l'opérateur de le conformer à l'anatomie du patient. Ce corps (1) est équipé à son extrémité considérée au niveau des volets de la valve, d'un embout profilé (2), en matériau souple, afin de traverser la valve et pré-dilater l'orifice central de la valve calcifiée afin d'autoriser le passage du corps (1) et de faciliter le positionnement de la zone de travail sans blesser les tissus sains. La deuxième fonction de cet embout est de pouvoir limiter la latéralisation de la partie ablative du dispositif vers l'anneau aortique. La décalcification de la valve procède avec une ablation tes tissus de l'orifice central vers la périphérie, jusqu'à pratiquer au contact de l'anneau aortique une décalcification profonde mais sans lésions des tissus de l'anneau aortique : l'embout en plastique qui sert à franchir la valve, une fois que le dispositif est situé au contact de l'anneau aortique, est déjà en appui au niveau tes tissus de la chambre de chasse du ventricule gauche et évite ainsi qu'une pression de la partie ablative du dispositif puisse se exercer au niveau de l'anneau aortique.

Cette extrémité équipée de l'embout (2), présente des agencements pour un débordement latéral d'un organe de coupe (3) desdits tissus. Comme cela sera indiqué dans la suite de la description, cet organe (3) est actionnable d'une seule main à partir d'une poignée (4) située à l'extérieur. Le corps (1) est monté libre en rotation par rapport à la poignée (4) en étant apte à être entraîné manuellement au moyen d'une molette (5). Les agencements pour le débordement latéral de l'organe de coupe (3), sont constitués par une ouverture (la), réalisée à l'extrémité du corps (1), au niveau de l'embout (2).

Selon une autre caractéristique de l'invention, l'extrémité du corps (1) présente, en amont de l'ouverture (la) pour le débordement latéral de l'organe de coupe (3), une zone arrondie (lb) en continuité avec ledit corps (1), mais déportée latéralement par rapport à ce dernier. Cette zone arrondie (lb), délimite du côté de l'ouverture (la), une partie en creux (1b1) et, à l'opposé, une partie débordante (lb2), apte à prendre appui avec les volets de la valve lors de l'opération de coupe sous un effet d'entraînement en rotation du corps (1), au moyen de la molette (5), pour réaliser la décalcification de toute la valve résultant d'un mouvement circulaire.

Dans une forme de réalisation, l'embout (2) ou l'extrémité du corps (1) au niveau dudit embout, est équipé d'un moyen de guidage réglable, apte à coopérer avec les tissus calcifiés, au fur et à mesure de l'opération d'ablation par le système de coupe (3), en combinaison avec un effet de trajectoire en spirale appliqué audit embout (2) ou audit corps.

L'ablation des tissus s'effectue selon une trajectoire en spirale partant du centre jusqu'à la périphérie de la valve par translation de rotation contre les bords des tissus par l'intermédiaire du système de coupe (3). La décalcification est effectuée en appliquant une pression latérale du système de coupe (3) en combinaison avec le moyen de guidage (5) sur les tissus calcifiés.

Le moyen de guidage est, par exemple, constitué par un ruban souple (8) apte à être déployé d'une manière circulaire en position de contact avec les tissus calcifiés. Avantageusement, le ruban (8) est déployé de manière excentrique par rapport à l'embout (2). Par exemple, le ruban (8) est monté en combinaison, d'une part avec un arbre rotatif commandé par un organe de manoeuvre accessible à partir de l'extérieur du corps (1), et, d'autre part, avec une partie fixe de l'embout (2) à partir de laquelle est déployé ledit ruban. Plus particulièrement, l'une des extrémités du ruban (8) est fixée à l'arbre rotatif pour être enroulée sur ce dernier et déborder au travers d'une ouverture que présente l'embout (2) ou le corps (1) afin d'être fixée par son autre extrémité sur la partie fixe dudit embout. Cette partie fixe peut être constituée par une fente afin de permettre audit ruban de déborder d'une manière excentrée. Dans cette forme de réalisation, la zone arrondie déportée latéralement (lb) du corps (1) n'est plus nécessaire.

Il en résulte que l'organe de coupe (3), en combinaison avec la zone arrondie (lb), permet la découpe du tissu par translation et rotation contre le bord de l'ouverture, depuis le centre jusqu'à la périphérie de la valve sous un effet de pression latérale.

L'organe de coupe (3) peut être constitué par une lame, un poinçon, une fraise, un système à ultra-sons,.... et peut présenter successivement plusieurs zones actives de différentes caractéristiques pour réaliser, en premier lieu, une coupe grossière, suivie par une coupe fine ou par des coupes de plus en plus fines.

Selon une autre caractéristique importante de l'invention, les organes de coupe (3), au niveau de l'ouverture (la) du corps (1), sont en communication avec des moyens d'aspiration (6) des tissus coupés. Plus particulièrement, la zone de coupe est combinée avec un système de lavage par aspiration, apte à enlever les débris des tissus de calcification dès leur ablation, afin d'éviter que ces derniers, en totalité ou en partie, ne se retrouvent dans le système sanguin circulatoire. Le système est mis en fonctionnement lorsque l'organe de coupe (3) est actionné et obture l'ouverture (la) afin de créer une étanchéité vis-à-vis de l'extérieur. De même, l'embout (2) présente latéralement un orifice (2a) relié à un conduit d'aspiration (6a). Ces dispositions évitent de sortir le dispositif de la racine aortique entre chaque découpe avant d'avoir terminé la décalcification complète de la valve.

Etant donné que l'intervention chirurgicale s'effectue par voie endoscopique, il est nécessaire de pouvoir visualiser la zone où l'intervention doit avoir lieu. Dans ce but, le dispositif est équipé d'un système de vision (7) débouchant dans la partie en creux (1b1) de la zone arrondie (lb) au niveau de l'organe de coupe (3). Ce système de vision (7) est de tout type connu et approprié et conforme à ceux couramment utilisés dans le domaine de la chirurgie endoscopique.

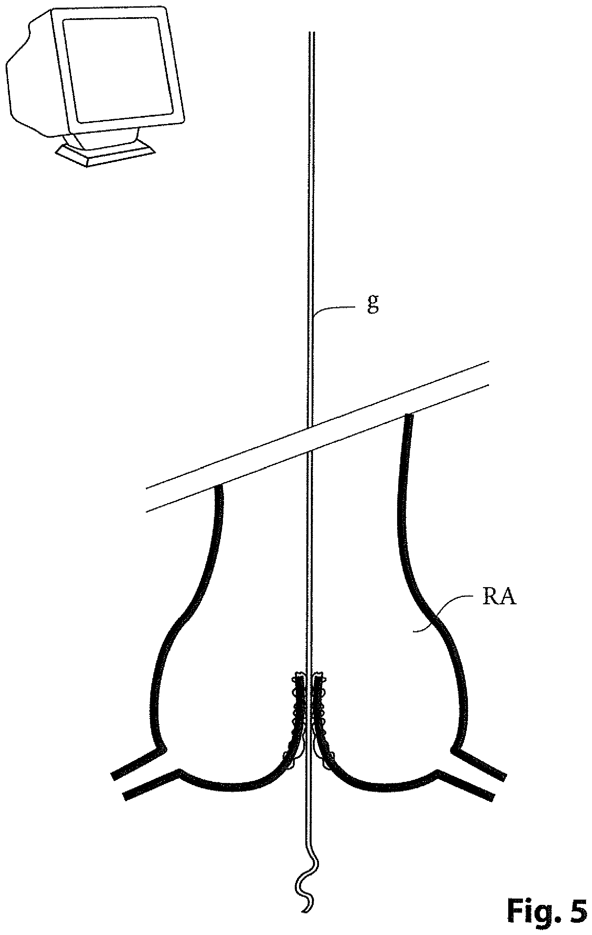

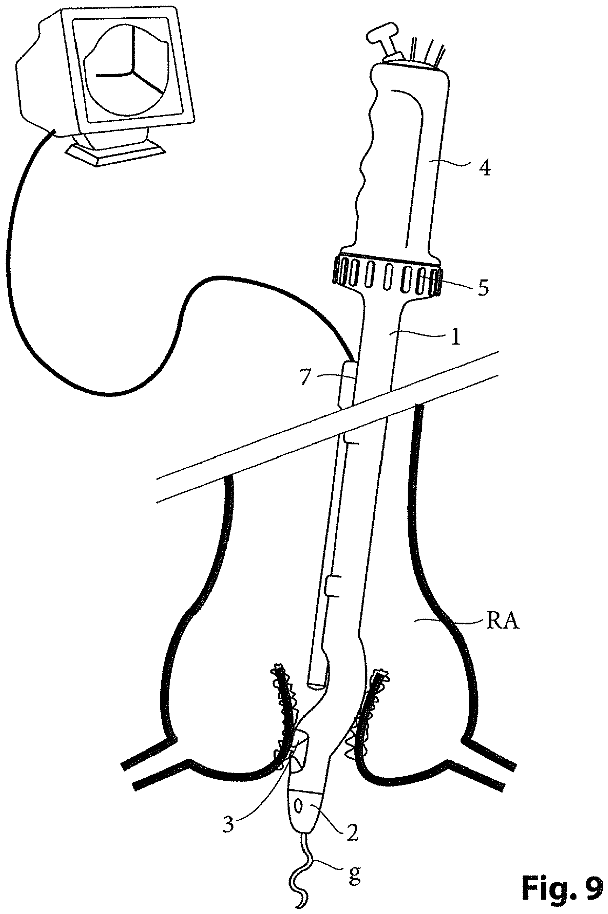

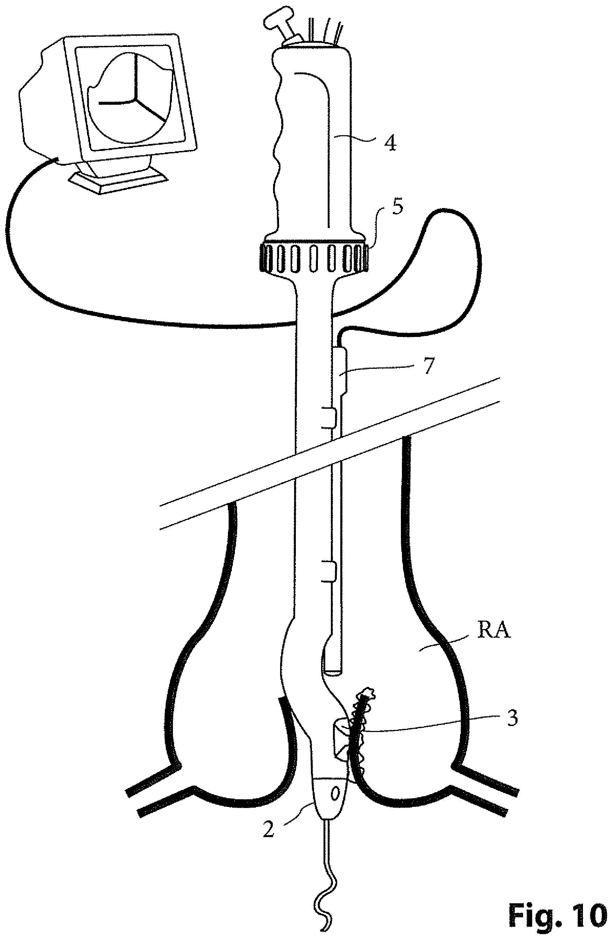

On se réfère aux figures 5 à 11 qui montrent les différentes séquences pour la mise en place du dispositif en vue de l'ablation des tissus calcifiés au niveau de la valve.

La figure 5 montre la racine aortique (RA) après mise en place du guide-fil (g), qui, de manière connue, est engagé afin de traverser la valve aortique sténosée.

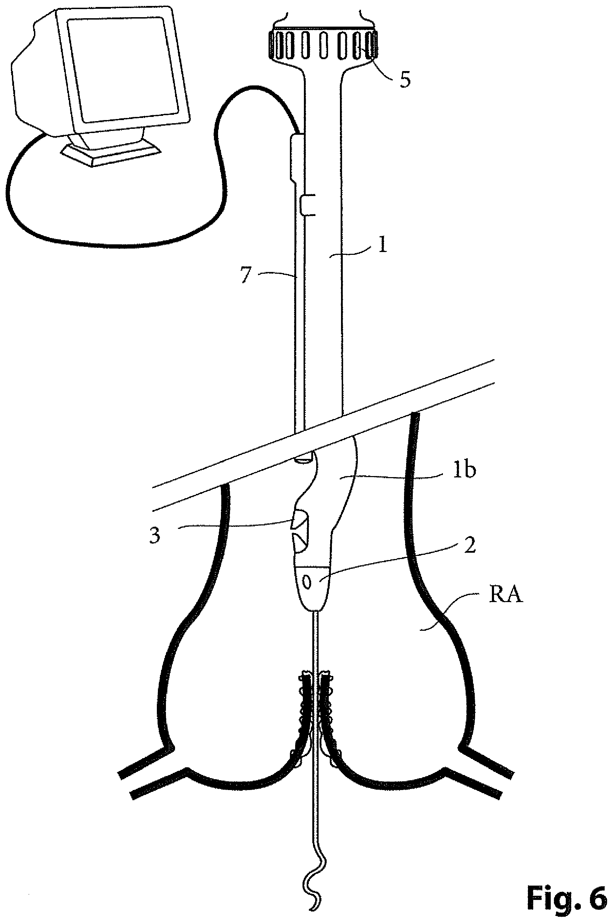

Le dispositif équipé du système de vision (7) est ensuite introduit sur le guide-fil (g) au-dessus de la partie de la valve où doit être enlevée la calcification (figure 6).

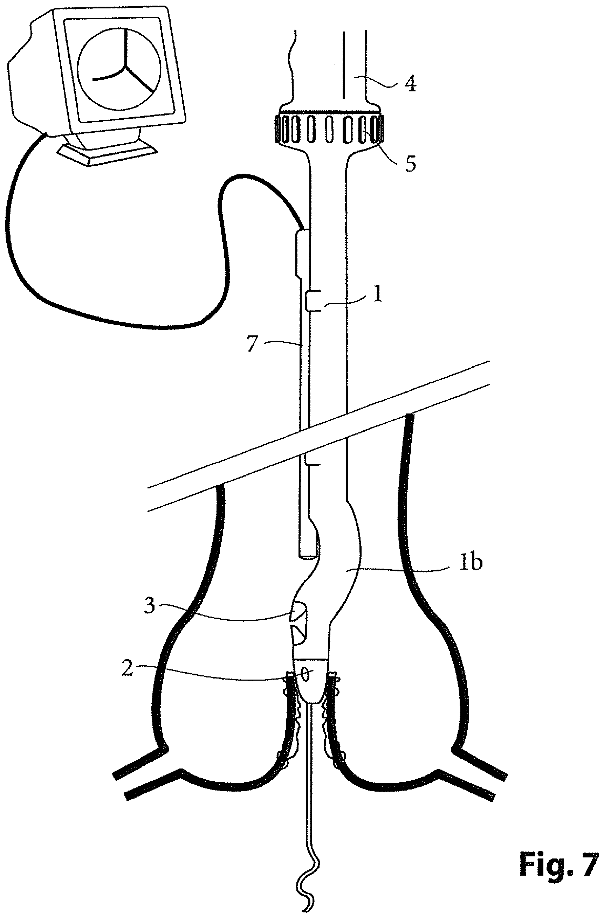

La figure 7 montre le dispositif en position d'engagement de l'embout (2) dans la zone calcifiée du volet.

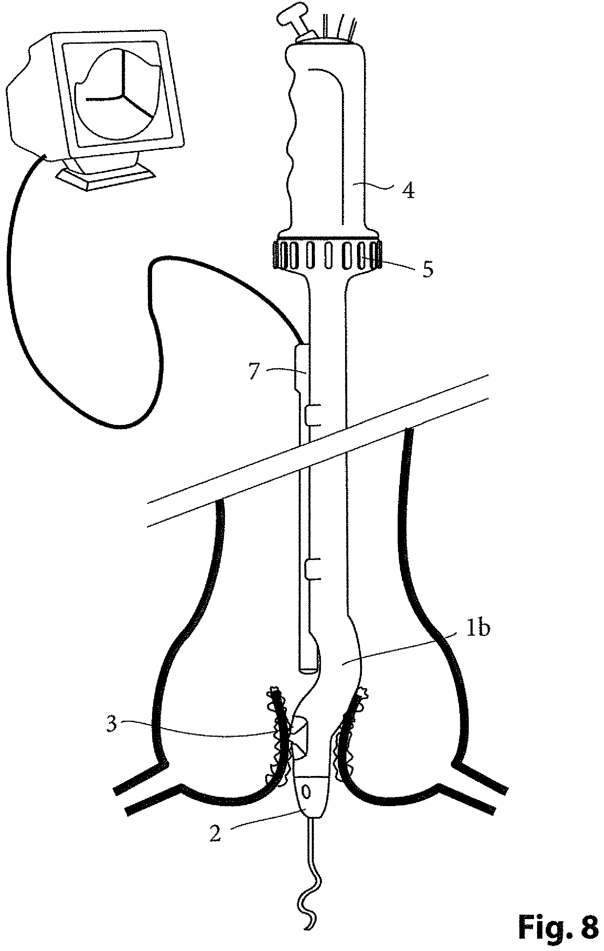

Les figures 8, 9 et 10 montrent l'opération d'ablation en tant que telle, des tissus calcifiés après actionnement de l'organe de coupe (3) et entraînement en rotation du corps (1) par rapport à la poignée (4), du centre jusqu'à la périphérie de la valve sous un effet de pression latérale. Comme indiqué, chaque fois que le dispositif de coupe est actionné, le système de lavage par aspiration est mis en fonctionnement.

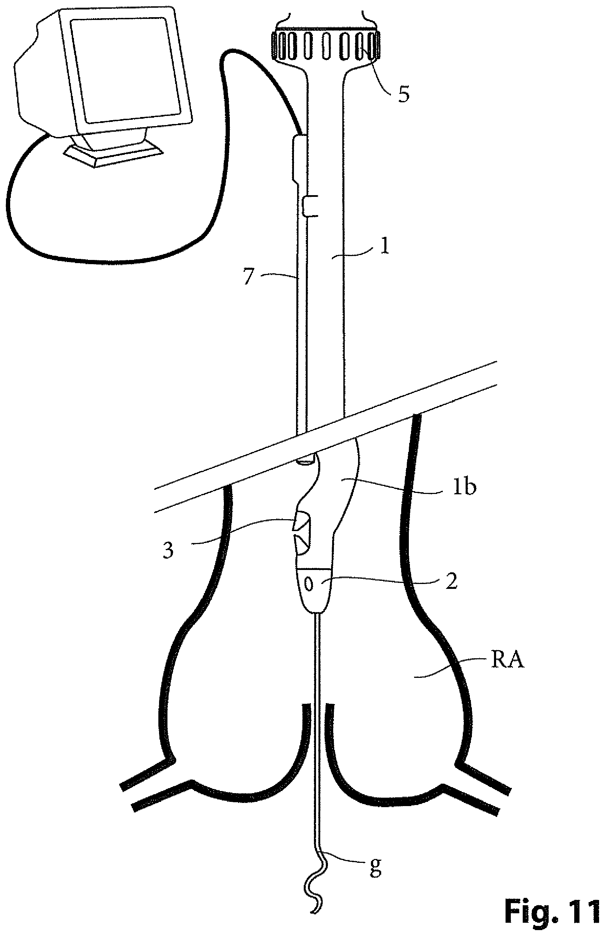

La figure 11 montre le retrait du dispositif après avoir terminé l'opération de décalcification. Il est alors possible de mettre en place une valve prothétique par tous moyens connus et appropriés.

Les avantages ressortent bien de la description.

## Revendications

1. Dispositif de découpe et d'évacuation de tissus calcifiés d'une valve cardiaque, comprenant un corps tubulaire (1) adapté pour être introduit par voie mini invasive ou endoscopique en relation avec un guide-fil (g) préalablement introduit et apte à traverser les volets de la valve au-dessus de la partie où doivent être enlevés les tissus calcifiés, l'une des extrémités dudit corps (1) considérée au niveau desdits volets, présentant un embout profilé (2) en matériau souple et des agencements (la) pour un débordement latéral d'un organe de coupe (3) desdits tissus, actionnable d'une seule main à partir d'une poignée (4) située à l'extérieur, lesdits agencements (la) étant en communication avec des moyens (6) d'aspiration des tissus coupés, **caractérisé en ce que**
l'extrémité du corps (1) présente en amont, des agencements (la) pour le débordement latéral de l'organe de coupe (3), une zone arrondie (1b) déportée latéralement pour délimiter, du côté des agencements (la), une partie en creux (1b1), et à l'opposé, une partie débordante (lb2) apte à prendre appui avec les volets lors de l'opération de coupe sous un effet d'entraînement en rotation du corps (1) pour réaliser la décalcification de toute la valve.

2. Dispositif selon la revendication 1, ***caractérisé* en ce que** l'embout (2) ou l'extrémité du corps (1) au niveau du raccordement avec ledit embout, est équipé d'un moyen de guidage réglable apte à coopérer avec les tissus calcifiés au fur et à mesure de l'opération d'ablation par le système de coupe (3) en combinaison avec un effet de trajectoire en spirale appliqué à l'embout.

3. Dispositif selon la revendication 2, ***caractérisé* en ce que** le moyen de guidage réglable est un ruban souple apte à être déployé d'une manière circulaire et excentrée en position de contact avec les tissus calcifiés.

4. Dispositif selon la revendication1, ***caractérisé* en ce que** le corps (1) est monté libre en rotation par rapport à la poignée (4) en étant apte à être entraîné manuellement au moyen d'une molette (5).

5. Dispositif selon l'une quelconque des revendications 1 à4, ***caractérisé* en ce qu'**il présente un système de vision (7) au niveau de l'organe de coupe (3).

6. Dispositif selon l'une quelconque des revendications 1 à5, ***caractérisé* en ce que** le corps (1) présente un moyen apte à maintenir le système de vision (7) en position stable pendant l'opération de coupe.

7. Dispositif selon la revendication 1, ***caractérisé* en ce que** les agencements pour le débordement latéral de l'organe de coupe (3) sont constitués par une ouverture (la) formée à l'extrémité du corps, au niveau de l'embout (2).

8. Dispositif selon la revendication 1, ***caractérisé* en ce que** l'organe de coupe (3) est conformé pour découper les tissus par translation et/ou rotation contre le bord de l'ouverture, depuis le centre jusqu'à la périphérie de la valve, sous un effet de pression latérale.

9. Dispositif selon la revendication8, ***caractérisé* en ce que** l'organe de coupe (3) présente successivement des zones actives de différentes caractéristiques, pour réaliser, d'abord une coupe grossière, puis, une ou des coupe(s) fine(s).

10. Dispositif selon l'une quelconque des revendications 1 à9, ***caractérisé* en ce qu'**il est combiné à un système de lavage/aspiration apte à enlever les débris des tissus et de calcification dès leur ablation, ledit système étant mis en fonctionnement lorsque l'organe de coupe (3) est actionné et obstrue l'ouverture afin de créer une étanchéité vis-à-vis de l'extérieur.

11. Dispositif selon la revendication 10, ***caractérisé* en ce que** l'embout (2) présente latéralement un orifice en communication avec un conduit d'aspiration.

12. Dispositif selon la revendication 1, ***caractérisé* en ce que** l'embout souple (2) est réalisé dans une couleur différente de la zone à décalcifier et présente une graduation pour un positionnement précis de la zone de travail présentant l'organe de coupe (3).

## Patentansprüche

1. Vorrichtung zum Herausschneiden und Entfernen kalzifizierter Gewebe aus einer Herzklappe, wobei sie einen röhrenförmigen Körper (1) umfasst, der dafür geeignet ist, auf minimalinvasivem oder endoskopischem Weg in Verbindung mit einer Fadenführung (g) eingeführt zu werden, welche im Vorfeld eingeführt wurde und dazu befähigt ist, durch die Segel der Klappe zu gelangen, die sich oberhalb des Abschnitts befindet, in welchem kalzifizierte Gewebe entfernt werden müssen, wobei ein Ende des Körpers (1), wenn es auf Höhe der Segel betrachtet wird, ein Profilendstück (2) aus nachgiebigem Material aufweist sowie Aussparungen (1a) für ein seitliches Hervorstehen eines Organs (3) zum Schneiden der Gewebe, welches mit einer Hand ausgehend von einem außengelegenen Griffstück (4) betätigt werden kann, wobei die Aussparungen (1a) mit Mitteln (6) zum Absaugen der geschnittenen Gewebe in Verbindung stehen, **dadurch gekennzeichnet, dass** das Ende des Körpers (1) vor den Aussparungen (1a) für das seitliche Hervorstehen des Schneideorgans (3) einen abgerundeten, seitlich versetzten Bereich (lb) aufweist, um auf der Seite der Aussparungen (la) einen Hohlabschnitt (1b1) und, auf der gegenüberliegenden Seite, einen hervorstehenden Abschnitt (lb2) abzugrenzen, wobei letzterer dazu befähigt ist, unter Einwirkung der Drehbewegung, in welche der Körper (1) versetzt wird, während des Schneidevorgangs auf den Segeln aufzuliegen, um die gesamte Klappe zu entkalzifizieren.

2. Vorrichtung nach Anspruch 1, **dadurch gekennzeichnet, dass** das Endstück (2), oder das Ende des Körpers (1) auf Höhe der Verbindung mit diesem Endstück, mit einem einstellbaren Führungsmittel versehen ist, das dazu befähigt ist, in Kombination mit einer spiralförmigen Wegstrecke, gemäß welcher das Endstück zur Anwendung gebracht wird, im Laufe des Vorgangs des Abschälens durch das Schneidesystem (3) mit den kalzifizierten Geweben zusammenzuwirken.

3. Vorrichtung nach Anspruch 2, **dadurch gekennzeichnet, dass** es sich bei dem einstellbaren Führungsmittel um ein nachgiebiges Band handelt, das dazu befähigt ist, kreisend und exzentriert derart positioniert zu werden, dass es sich mit den kalzifizierten Geweben in Kontakt befindet.

4. Vorrichtung nach Anspruch 1, **dadurch gekennzeichnet, dass** der Körper (1) frei drehbar gegenüber dem Griffstück (4) angebracht ist, wobei er dazu befähigt ist, mittels eines Stellrads (5) von Hand angetrieben zu werden.

5. Vorrichtung nach einem beliebigen der Ansprüche 1 bis 4, **dadurch gekennzeichnet, dass** sie ein Sichtsystem (7) auf Höhe des Schneideorgans (3) aufweist.

6. Vorrichtung nach einem beliebigen der Ansprüche 1 bis 5, **dadurch gekennzeichnet, dass** der Körper ein Mittel (1) aufweist, das dazu befähigt ist, das Sichtsystem (7) während des Schneidevorgangs in stabiler Position zu halten.

7. Vorrichtung nach Anspruch 1, **dadurch gekennzeichnet, dass** die Aussparungen für das seitliche Hervorstehen des Schneideorgans (3) aus einer Öffnung (1a) bestehen, die am Ende des Körpers auf Höhe des Endstücks (2) ausgebildet ist.

8. Vorrichtung nach Anspruch 1, **dadurch gekennzeichnet, dass** das Schneideorgan (3) derart ausgestaltet ist, dass die Gewebe durch eine Translations- und/oder Drehbewegung gegen den Rand der Öffnung herausgeschnitten werden, ausgehend von der Mitte bis zum Randbereich der Klappe, unter Einwirkung eines seitlichen Drucks.

9. Vorrichtung nach Anspruch 8, **dadurch gekennzeichnet, dass** das Schneideorgan (3) nacheinander Wirkbereiche mit verschiedenen Eigenschaften aufweist, um zunächst einen groben Schnitt und dann einen oder mehrere feine(n) Schnitt(e) durchzuführen.

10. Vorrichtung nach einem beliebigen der Ansprüche 1 bis 9, **dadurch gekennzeichnet, dass** sie mit einem System zum Waschen/Absaugen kombiniert ist, welches dazu befähigt ist, Gewebe- und Kalzifizierungsbruchstücke zu entfernen, sobald diese abgeschält wurden, wobei das System in Betrieb gesetzt wird, wenn das Schneideorgan (3) betätigt wird, und es die Öffnung verschließt, um eine Abdichtung nach außen hin zu bewirken.

11. Vorrichtung nach Anspruch 10, **dadurch gekennzeichnet, dass** das Endstück (2) seitlich eine Öffnung aufweist, die mit einer Saugleitung in Verbindung steht.

12. Vorrichtung nach Anspruch 1, **dadurch gekennzeichnet, dass** das nachgiebige Endstück (2) derart hergestellt ist, dass es eine andere Farbe als der zu entkalzifizierende Bereich hat, und mit einer Messeinteilung versehen ist, um den Arbeitsbereich, welcher das Schneideorgan (3) aufweist, genau zu positionieren.

## Claims

1. A device for cutting off and removing calcified tissue from a heart valve, comprising a tubular body (1) suitable for being inserted by means of a minimally invasive or endoscopic channel in relation to a previously inserted guide wire (g) and suitable for passing through the flaps of the valve above the portion where the calcified tissue is to be removed, one of the ends of said body (1) viewed at the level of said flaps, having a profiled tip (2) made of flexible material and arrangements (la) for the lateral projection of a cutting member (3) for cutting said tissue, that can be actuated using only one hand by means of a handle (4) located externally, said arrangements (la) being in communication with means (6) for sucking away the cut tissue **characterized in that** the end of the body (1) having, upstream from the arrangements (1a) for the lateral projection of the cutting member (3), a laterally offset rounded area (1b) defining, on the same side as the arrangements (1a), a recessed portion (1b1) and, on the opposite side, a projecting portion (1b2) suitable for bearing upon the flaps during the cutting procedure under the effect of a rotational movement of the body (1), in order to decalcify the whole valve .

2. The device according to claim 1, ***characterized* in that** the tip (2) or the end of the body (1) at the connection with said tip, is equipped with an adjustable guide means suitable for gradually engaging with calcified tissue during the ablation procedure using the cutting system (3) in combination with a spiral trajectory effect applied to the tip.

3. The device according to claim 2, ***characterized* in that** the adjustable guide means is elastic tape suitable for being deployed in a circular and eccentric manner in a position of contact with the calcified tissue.

4. The device according to claim 1, ***characterized* in that** the body (1) is mounted free to rotate relative to the handle (4) such that it is capable of being driven manually by means of a knob wheel (5).

5. The device according to any of claims 1 to 4, ***characterized* in that** it has a vision system (7) at the level of the cutting member (3).

6. The device according to any of claims 1 to 5, ***characterized* in that** the body (1) has a means of maintaining the vision system (7) in a stable position during the cutting procedure.

7. The device according to claim 1, ***characterized* in that** the arrangements for the lateral projection of the cutting member (3) consist of an opening (la) formed at the end of the body, at the tip (2).

8. The device according to claim 1, ***characterized* in that** the cutting member (3) conforms to the cutting of tissue by means of translation and/or rotation against the edge of the opening, from the center to the periphery of the valve, as a result of lateral pressure.

9. The device according to claim 8, ***characterized* in that** the cutting member (3) has successive active areas with different features, in order to achieve, first a coarse cut, then one or more fine cuts.

10. The device according to any of claims 1 to 9, ***characterized* in that** it is combined with a washing/suction system that is suitable for removing tissue and calcification debris upon the ablation thereof, said system being put into operation when the cutting member (3) is actuated, and obstructing the opening in order to create a seal with respect to the exterior thereof.

11. The device according to claim 10, ***characterized* in that** the tip (2) has a lateral opening that is in communication with a suction line.

12. The device according to claim 1, ***characterized* in that** the flexible tip (2) is made using a different color to that of the decalcifying area and has a graduation for the precise positioning of the working area with the cutting member (3).
